(19) Europäisches Patentamt
European Patent Office
Office européen des brevets

(11) EP 1 452 855 A1

(12) **EUROPEAN PATENT APPLICATION**
published in accordance with Art. 158(3) EPC

(43) Date of publication:
**01.09.2004 Bulletin 2004/36**

(21) Application number: **02785934.7**

(22) Date of filing: **13.11.2002**

(51) Int Cl.7: **G01N 27/327**

(86) International application number:
**PCT/JP2002/011819**

(87) International publication number:
**WO 2003/042681 (22.05.2003 Gazette 2003/21)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **14.11.2001 JP 2001349066**
**20.06.2002 JP 2002180363**

(71) Applicants:
- **MATSUSHITA ELECTRIC INDUSTRIAL CO., LTD.**
  **Kadoma-shi, Osaka 571-8501 (JP)**
- **MATSUSHITA REFRIGERATION COMPANY**
  **Kusatsu-shi, Shiga 525-8555 (JP)**

(72) Inventors:
- **HATTORI, Akiyoshi**
  **Yawata-shi, Kyoto 614-8297 (JP)**
- **MACHII, Nobuyuki**
  **Komagane-shi, Nagano 399-4117 (JP)**
- **HORI, Yoshihiro**
  **Ikoma-shi, Nara 630-0141 (JP)**
- **WAKITA, Katsuya**
  **Nara-shi, Nara 631-0074 (JP)**
- **SASABE, Shigeru**
  **Otsu-shi, Shiga 520-2144 (JP)**
- **AKEYAMA, Yukako**
  **Ikoma-shi, Nara 630-0121 (JP)**
- **WADA, Tomoko**
  **Yamaguchi 752-0976 (JP)**

(74) Representative: **Grünecker, Kinkeldey,**
**Stockmair & Schwanhäusser Anwaltssozietät**
**Maximilianstrasse 58**
**80538 München (DE)**

# (54) GAS SENSOR, AND PRODUCTION METHOD FOR GAS SENSOR

(57) The judgment about the freshness of food and drink has been made subjectively, and hence often not clear-cut; therefore, investigation has been actively made of the technology to use a semiconductor gas sensor to sense the freshness of food and drink. However, the gas sensors of the prior art have not been capable of sensing gases of, for example, ethylene, ethanol, aldehydes, mercaptans and amines, which are important in sensing the freshness of vegetables and fruits, with satisfactory responsiveness and excellent durability.

The gas sensor of the present invention includes: an insulating substrate 1; a pair of thin film electrodes 2 provided on the insulating substrate 1 in such a manner as to leave a fixed space between them; a gas sensitive film 3 including a metallic oxide which is provided in such a manner as to substantially fill at least the fixed space left between the electrodes; and a catalytically active protective layer 5 formed in such a manner as to cover the surface of the metallic oxide exposed to the outside.

Fig. 1

3
5
1
2

EP 1 452 855 A1

## Description

### Technical Field

The present invention relates to a gas sensor and a method of producing the same for sensing the freshness or decay of, for example, vegetables and fruits.

### Background Art

The judgment about the freshness of food and drink has been made subj ectively based on the senses of persons, such as the sense of sight, the sense of taste or the sense of swallow, and hence often not clear-cut; therefore, investigation has been actively made of the technology to use a semiconductor gas sensor to sense the freshness of food and drink.

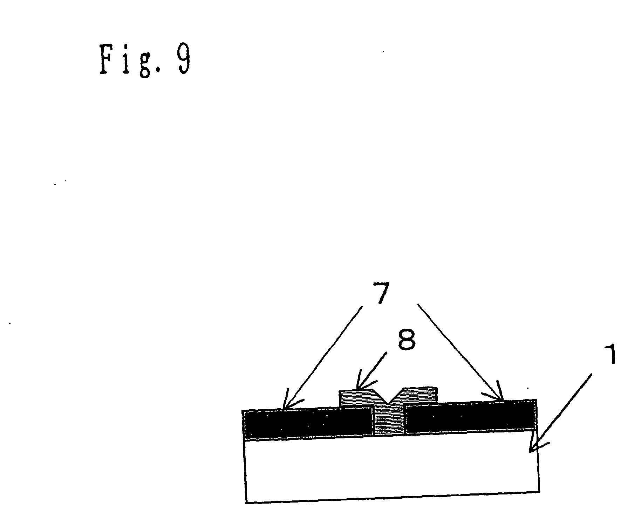

In general, semiconductor gas sensors are made up of: an insulating substrate 1, a pair of electrodes 7 and a gas sensitive body 8, as shown in Figure 9. The gas sensor of the Figure 9 is constructed in such a manner as to provide the pair of electrodes 7 on the insulating substrate 1 and form the gas sensitive body 8 on the insulating substrate 1 and the pair of electrodes 7.

In recent years, semiconductor gas sensors have been developed which are capable of sensing trimethylamine, an offensive odor component generated from raw fishes, and used to sense the freshness of the raw fishes. As a sensing material, titanium-dioxide oxide semiconductor has been widely used, and a metal component has been added as a catalyst to the titanium dioxide to improve the sensitivity of the sensors. In this case, it is the action and dispersing state of the catalyst and the thickness of the sensitive film that have effect on the sensitivity of the sensors, and the component and amount of the catalyst added play an important part in improving the catalytic action. Apart from using titanium dioxide as a sensing material, investigation has been made of improving the sensitivity of the sensors by using magnesium-added indium oxide as a sensing material for the gas sensors which sense trimethylamine and adding 5% by mol of magnesium oxide to the indium oxide to decrease the electron density by the atomic control so that the sensor resistance in the air is increased. However, these gas sensors, which sense trimethylamine, are still in the elementary stage of investigation and have not been put to use yet; moreover, their power consumption is too large and is not suitable for mass production.

As for the freshness-sensing devices of sensing the freshness of vegetables, unlike the sensors that sense trimethylamine generated from raw fishes, sensors of sensing freshness of vegetables have been developed which are excellently sensitive to sulfide gases (mercaptans) generated from vegetables. For example, there are disclosed methods of producing vegetable-freshness sensing sensors which include : a step of adding and mixing a predetermined amount of palladium powder to and with tin oxide powder and grinding the mixture; a step of calcining the grinded tin oxide and palladium powders at a specified temperature for a specified period of time and mixing the calcined powders with an organic matter into paste; a step of coating the electrode surfaces of a substrate with the paste to form a sensing film; and a step of drying the coating, firing the same at a specified temperature for a specified period of time and attaching lead wires to the electrode surfaces (see, for example, Japanese Patent No. 2875174).

The entire disclosure of Japanese Patent No. 2875174 specification is incorporated herein by reference in its entirety.

It has been found that slight amounts of ethylene, ethanol and aldehydes are generated from vegetables and fruits even in the early time of their freshness, mercaptans from the beginning of their decay and that amines such as ammonia are generated from fruits from the beginning of their decay. As a result, it has also been revealed that in sensing the freshness of vegetables and fruits, it is effective to sense gases of ethylene, ethanol and aldehydes, whereas in sensing the decay of vegetables and fruits, it is effective to sense gases of mercaptans and amines such as ammonia.

However,for the vegetable-freshnesssensingsensors of the prior art described above that include a sensing film formed in the steps of : mixing a predetermined amount of palladium powder with tin oxide powder, grinding the mixture, calcining the grinded tin oxide and palladium powders, and mixing the calcined powders with an organic matter into paste; coating the electrode surfaces of a substrate with the paste ; and drying and f iring the coating, it is difficult to sense gases of ethylene, ethanol and aldehydes at a 1 ppm level, which should be effectively used in sensing the freshness of vegetables, with satisfactory responsiveness (with high sensitivity). It is also difficult to sense gases of mercaptans and amines at a level of 1 ppm, which should be effectively used in sensing the decay of vegetables and fruits.

Further, when using such a semiconductor-type of gas sensor on a refrigerator to sense the freshness or decay of vegetables and fruits, after long-term use of the refrigerator, the gas sensor deteriorates and its output signal is weakened, which sometimes prevents the sensor from sensing the freshness and decay. The present inventors attribute the weakening (or the deterioration) of the output of the semiconductor type sensor to the deterioration with time of its electrodes and catalyst, which play a key role of the sensor, with the progress of the sensing reaction, and think that the sensing reaction of gases, as objects of sensing, is inhibited due to the reduction of the catalyst by the reducing gases of alcohol, aldehydes, etc. generated from vegetables and fruits or the rigid adsorption of mercaptans and amines generated from the same on the electrode surface and the catalyst. In the semiconductor-type of gas sensors, though noble metals are often used for their electrodes and catalysts, which have the key function of sensing, the noble metals are poorly resistant to sulfur-based compounds and silicone-based compounds, and hence easy to deteriorate. This sometimes makes it very difficult to ensure the durability (reliability) of such gas sensors.

Thus, with the gas sensors of the prior art, it is difficult to sense gases of ethylene, ethanol, aldehydes, mercaptans, amines, etc. with satisfactory responsiveness and excellent durability.

Disclosure of the Invention

In light of the above-described problems the prior art has, the obj ect of the present invention is to provide a gas sensor capable of sensing gases of ethylene, ethanol, aldehydes, mercaptans and amines, which are important in sensing the freshness of vegetables and fruits, with satisfactory responsiveness and excellent durability, for example.

A first invention of the present invention is a gas sensor, comprising:

an insulating substrate (1);

a pair of first electrodes (2) provided on the substrate (1) in such a manner as to leave a fixed space between them;

a gas sensitive body (3) including a metallic oxide, which is provided in such a manner as to substantially fill at least the fixed space; and

a catalytically active protective layer (5) formed in such a manner as to cover the surface of the metallic oxide exposed to the outside and/or to make up for the gaps exiting inside the metallic oxide.

A second invention of the present invention is the gas sensor according to the first invention of the present invention, wherein the first electrodes (2) are thin film electrodes (2),

further comprising a pair of thick film electrodes (6) for applying a voltage, provided on the respective thin film electrodes (2).

A third invention of the present invention is the gas sensor according to the second invention of the present invention, wherein the thick film electrodes (6) are provided in such a manner as not to come in contact with the gas sensitive body (3).

A fourth invention of the present invention is the gas sensor according to the third invention of the present invention, wherein the catalytically active protective layer (5) is formed in such a manner as to also cover the thick film electrodes (6).

A fifth invention of the present invention is the gas sensor according to the first invention of the present invention, wherein the metallic oxide contains, as a main constituent, at least one of tin oxide, indium oxide, zinc oxide and tungsten oxide.

A sixth invention of the present invention is the gas sensor according to the first invention of the present invention, wherein the catalytically active protective layer (5) contains silicon oxide as a main constituent and at least one of titanium, vanadium, chromium, manganese, iron, cobalt, nickel, zinc, copper, platinum, palladium, ruthenium and rhodium, as a promoter.

A seventh invention of the present invention is a method of producing the gas sensor according to the first invention of the present invention, comprising a step of forming the catalytically active protective layer (5) using the sol-gel process.

An eighth invention of the present invention is a method of producing the gas sensor according to the first invention of the present invention, comprising a step of forming the catalytically active protective layer (5) using a solution obtained by: dropping and mixing an organic solution containing a promoter, water and an acid into an organic solution containing an organosilicon compound; and hydrolyzing and polycondensing.

A ninth invention of the present invention is the method of producing a gas sensor according to the eighth invention of the present invention, wherein the organosilicon compound contains at least one of tetraalkoxysilane, fluoroalkyltrialkoxysilane and difluoroalkyldialkoxysilane.

A tenth invention of the present invention is the method of producing a gas sensor according to the eighth invention of the present invention, wherein the promoter is any one of nitrate complex salts, chloride complex salts, sulfate complex salts and acetylacetonato complex salts which contains at least one metal of titanium, vanadium, chromium, manganese, iron, cobalt, nickel, zinc, copper, platinum, palladium, ruthenium and rhodium.

An eleventh invention of the present invention is the method of producing a gas sensor according to the eighth invention of the present invention, wherein the acid is any one of hydrochloric acid, sulfuric acid, nitric acid, ammonia and alcohol amines.

A twelfth invention of the present invention is the method of producing a gas sensor according to the eighth invention of the present invention, wherein the organic solution containing the organosilicon compound further contains an organometallic compound for accelerating the polycondensation.

A thirteenth invention of the present invention is the method of producing a gas sensor according to the twelfth invention of the present invention, wherein the organometallic compound is metallic alkoxide containing at least one metal of titanium, zirconium, tin, aluminum and zinc.

Brief Description of the Drawings

Figure 1 is a schematic cross-sectional view of a gas sensor (1) in accordance with the first embodiment of the present invention;
Figure 2 is a schematic cross-sectional view of a gas sensor (2) in accordance with the first embodiment of the present invention;
Figure 3 is a graph showing the relation between the sensitivity to dimethyl sulfide of the gas sensors of example 1 of the present invention and the element temperature;
Figure 4 is a graph showing the sensitivity to dimethyl sulfide of the gas sensors of example 2 of the present invention;
Figure 5 is a schematic cross-sectional view of a gas sensor (1) in accordance with the second embodiment of the present invention;
Figure 6 is a schematic cross-sectional view of a gas sensor (2) in accordance with the second embodiment of the present invention;
Figure 7 is a graph showing the responsive characteristics to dimethyl sulfide of the gas sensor of example 4 (5- to 15-fold dilutions) and the gas sensor without a catalytically active protective layer;
Figure 8 is graph showing the variation in resistance of sensor elements, the gas sensors'of example 5 of the present invention, in high-temperature and high-humidity test conducted at 40°C and 95% RH; and
Figure 9 is a schematic cross-sectional view of a conventional semiconductor-type gas sensor.

(Description of Symbols)

1 Insulating Substrate
2 Thin Film Electrodes
3 Gas sensitive film
5 Catalytically Active Protective Layer
6 Thick Film Electrodes
13 Gas sensitive film
14 Metallic Oxide Thin Film
15 Catalytically Active Protective Layer

Best Mode for Carrying Out the Invention

In the following the embodiments of the present invention will be described with reference to the accompanying drawings.

(Embodiment 1)

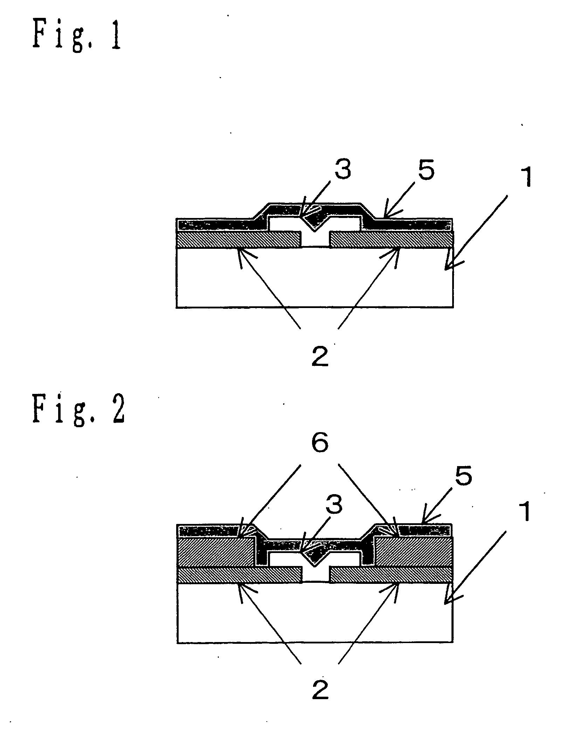

First, the construction and operation of gas sensors in accordance with this embodiment will be described with reference mainly to Figures 1 and 2.

Figures 1 and 2 are schematic cross-sectional views of gas sensors in accordance with embodiment 1 of the present invention.

Reference numeral 1 denotes an insulating substrate of alumina, mullite, or the like, reference numeral 2 thin film electrodes made up of a metal such as gold, silver or platinum, reference numeral 3 a gas sensitive film made up of metallic oxide whose main constituent is tin oxide, indium oxide, zinc oxide, tungsten oxide, or the like, reference numeral 5 a catalytically active protective layer whose main constituent is silicon oxide, and reference numeral 6 thick electrodes made up of a metal such as gold, silver or platinum.

As the substrate 1, any substrate can be used as long as its surface is electrically insulating and it has a heating function, and its material and construction are not limited to any specific ones. However, the surface roughness of the substrate is preferably in the range of 0.01 to 1 μm.

The purpose of the thin film electrodes 2 and the thick film electrodes 6 is to apply a voltage to the gas sensitive body 3 so that its resistance is measured, and their material, construction, pattern and production method are not limited to any specific ones. However, preferably the thickness of the thin film electrodes 2 is in the range of 0.1 to 1 μm and that of the thick film electrodes 4 in the range of 3 to 20 μm. The thick film electrodes 4 are for better electrodes' joining to lead wires, and they are not necessarily needed from the viewpoint of the gas sensor's construction.

The insulating substrate 1 is equivalent to the substrate of the present invention, the thin film electrodes 2 to first electrodes of the present invention, the gas sensitive film 3 to the gas sensitive body of the present invention, and the catalytically active protective layer 5 to the catalytically active protective layer of the present invention. Further, the thin film electrodes 2 are equivalent to the thin film electrodes of the present invention and the thick film electrodes 6 to the thick film electrodes of the present invention.

Then the method of producing the gas sensors in accordance with this embodiment will be described.

The gas sensitive film 3 can be formed in the following manner.

The gas sensitive film 3 is formed by forming a film of a composition for forming the gas sensitive thin film 3 on the substrate and firing the formed film at a temperature of several hundred degrees or higher. For the application of the composition for forming the gas sensitive body, methods such as screen coating, roll coating, dip coating and spin coating can be used; however, the screen coating method is preferable. As the firing temperature, any temperature can be used as long as it is not less than the temperature which permits the decomposition of the composition for forming the gas sensitive film 3 and not more than the temperature which permits the deformation of the substrate 1, and preferably the temperature is in the range of 400 to 800°C.

The composition for forming the gas sensitive film 3 is synthesized in the following manner.

First, an organic solvent is added to an activator to dissolve it. The activator is a metallic salt that is added to improve the sensitivity and gas selectivity of the gas sensitive film 3, and concrete examples of the activators applicable are alkaline earth metallic salts such as metallic magnesium salts, metallic calcium salts, metallic strontium salts and metallic barium salts; transition metallic salts such as metallic titanium salts, metallic zirconium salts, metallic vanadium salts, metallic chromium salts, metallic manganese salts, metallic iron salts, metallic cobalt salts, metallic nickel salts and metallic copper salts; metallic zinc salts; metallic lead salts; metallic cadmium salts; metallic antimony salts; metallic bismuth salts ; and metallic palladium salts. As the metallic salt compound, any compound can be used as long as it is relatively stable at room temperature, but easily decomposed by heat treatment, and it does not matter whether it is an inorganic salt or an organic salt. The inorganic salts applicable include, for example, nitrates, sulfates and chlorides. And the organic salts applicable include, for example, carboxylates, dicarboxylates and acetylacetonato complex salts. As the organic solvent, any organic solvent can be used as long as it can dissolve both metallic tin soap and a viscosity modifier. The organic solvents applicable include, for example, ether alcohols such as methoxyethanol and butyl carbitol; β-diketones such as acetylacetone; esters such as butyl carbitol acetate; and terpene-based solvents such as α-terpineol.

Then, a viscosity modifier is added to and mixed with the above organic solution. As the viscosity modifier, any polymer can be used as long as it produces a thickening effect of increasing the viscosity of the organic solution. Such viscosity modifies include, for example, polyvinylpyrrolidinone and ethyl cellulose.

Lastly, metallic tin soap is added to and mixed with the above organic solution. The metallic tin soap applicable includes, for example, tin 2-ethylhexanoate and tin naphthenate.

As the gas sensitive film 3, any thin film can be used as long as it is a metallic oxide semiconductor thin film, and concrete examples of the metallic oxide semiconductor thin films are thin films of tin oxide, indium oxide, tungsten oxide and zinc oxide. The process of producing the thin film is not limited to thermal decomposition method of the aforementioned organometallic compounds, but chemical film forming processes, such as sol-gel process and CVD process, and physical film forming processes, such as vacuum evaporation coating process and sputtering process, can also be used.

The catalytically active protective layer 5 can be formed in the following manner.

The catalytically active protective layer 5 is formed by forming a film of a composition for forming the catalytically active protective layer 5 on the gas sensitive thin film 3 and firing the formed film at a temperature of several hundred degrees or higher. For the application of the composition for forming the catalytically active protective layer 5, methods such as screen coating, roll coating, dip coating and spin coating can be used; however, the dip coating or spin coating method is preferable. As the firing temperature, any temperature can be employed as long as it is not less than the temperature which permits the decomposition of the composition for forming the catalytically active protective layer 5 and not more than the temperature which permits the deformation of the substrate 1, and preferably the temperature is in the range of 400 to 800°C.

In more particular, the composition for forming the catalytically active protective layer 5 is synthesized in the following manner.

First, an organosilicon compound is added to and dissolved in an organic solvent to obtain a raw material solution.

As the organosilicon compound, any organosilicon compound can be used as long as it undergoes hydrolysis/polycondensation, and concrete examples of the organosilicon compounds applicable are tetraalkoxysilanes such as tetraethyl orthosilicate, fluoroalkyltrialkoxysilane such as fluoromethyltriethyl silicate, and metal alkoxide such as difluoroalkyldialkoxysilane. Then, a predetermined amount of water, as the hydrolysis solution, and an acid such as hydrochloric acid, nitric acid or sulfuric acid, as a catalyst for the hydrolysis, are dissolved in an organic solvent. A promoter can also be added to this organic solvent. The promoter is a metallic salt added to improve the catalytic activity of the catalytically active protective layer 5 and the reliability and gas selectivity of the gas sensitive film 3, and concrete examples of the promoters applicable are transition metallic salts such as metallic titanium salts, metallic vanadium salts, metallic chromium salts, metallic manganese salts, metallic iron salts, metallic cobalt salts, metallic nickel salts, metallic zinc salts, metallic copper salts, and noble metallic salts such as metallic platinum salts, metallic palladium salts, metallic ruthenium salts and metallic rhodium salts. As the compound, any compound can be used as long as it is dissolved in an acidic solution and exists stably, and it does not matter whether it is an inorganic salt or an organic salt. The inorganic salts applicable include, for example, nitrates, sulfates and chlorides. And the organic salts applicable include, for example, carboxylates, dicarboxylates and acetylacetonato complex salts. As the organic solvent, considering the reactivity with the alkoxyl groups of the organosilicon compound, organic solvents having the same functional groups, in particular, alcohols, such as methanol and ethanol, and ether alcohols, such as methoxyethanol and ethoxyethanol, can be used.

Lastly, a predetermined amount of water, acid, and, depending on the situation, hydrolysis solution containing a promoter are dropped into and mixed with the raw material solution in which the organosilicon compound has been dissolved to obtain a composition for forming the catalytically active protective layer 5. During or after the hydrolysis, heat may be applied.

The catalytically active protective layer 5 produces electrical charges when alcohols, aldehydes, mercaptans or amines are decomposed on its surface, and the charges moved from the catalytically active protective layer 5 to the gas sensitive film 3 produces apotential difference between the pair of thin film electrodes 2, which enables the sensing of gas concentration.

Specifically, in this embodiment, the catalytically active protective layer 5 senses alcohols, aldehydes, mercaptans or amines on its surface cooperatively with the gas sensitive film 3, whereby gases are prevented from directly coming in contact with the gas sensitive film 3. As a result, maintaining gas sensing capability of the sensor and protecting the gas sensitive thin film are allowed to be compatible with each other. If the catalytically active protective layer 5 contains a promoter, the sensitivity of the gas sensor to gas concentration is further improved.

In addition, since the catalytically active protective layer 5 is made a porous film, it allows part of alcohols, aldehydes, mercaptans or amines to pass through the pores of the film and reach the gas sensitive film 3, and thereby it contributes to sensing gas concentration. This is considered to further improve the sensitivity of the gas sensor. The gases of alcohols, aldehydes, mercaptans or amines having reached the thin film electrodes 2 and the gas sensitive film 3 may cause deterioration thereof; however, the amounts of such gases are kept extremely small, due to the catalytically active protective layer 5, compared with those in the gas sensors of the prior art. The catalytically active protective layer 5 thus produces the full effect of improving the durability of the sensor.

(Embodiment 2)

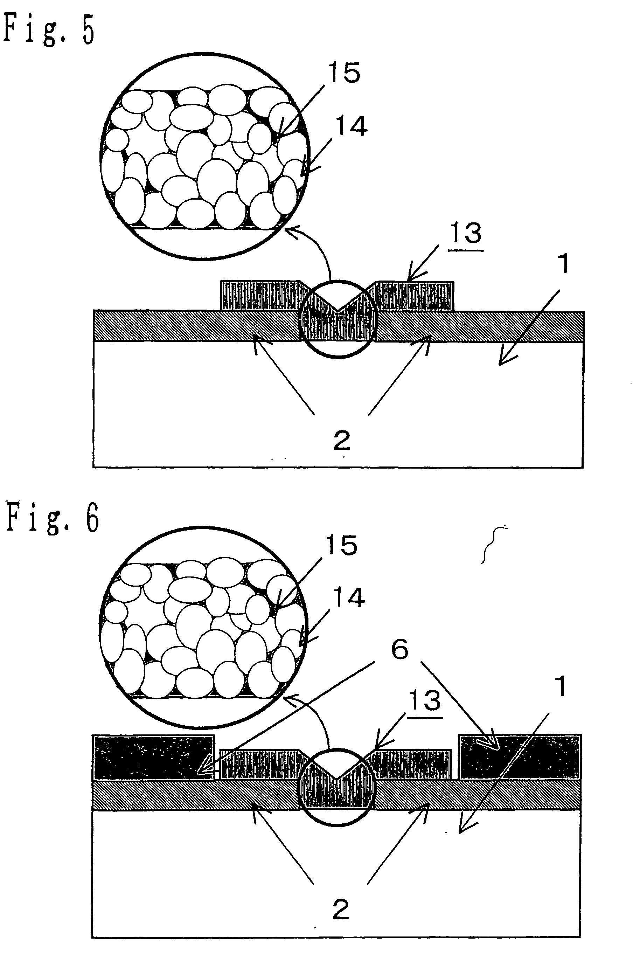

First, the construction and operation of gas sensors in accordance with this embodiment will be described with reference mainly to Figures 5 and 6.

Figures 5 and 6 are schematic cross-sectional views of gas sensors in accordance with embodiment 2 of the present invention.

Reference numeral 1 denotes an insulating substrate of alumina, mullite, or the like, reference numeral 2 thin film electrodes made up of a metal such as gold, silver or platinum, reference numeral 13 a gas sensitive film consisting of: a metallic oxide thin film 14 which has a network structure and contains tin oxide, indium oxide, zinc oxide, tungsten oxide, or the like as a main constituent; and a catalytically active protective layer 15 which is impregnated into the above metallic oxide thin film, contains silicon oxide as a main constituent, and has a water absorption rate of 5% or less, and reference numeral 6 thick electrodes made up of a metal such as gold, silver or platinum.

As the substrate 1, any substrate can be used as long as its surface is electrically insulating and it has a heating function, and its material and construction are not limited to any specific ones. However, the surface roughness of the substrate is preferably in the range of 0.01 to 1 μm. The purpose of the thin film electrodes 2 and the thick film electrodes 6 is to apply a voltage to the gas sensitive body 13 so that its resistance is measured, and their material, construction, pattern and production method are not limited to any specific ones. However, preferably the thickness of the thin film electrodes 2 is in the range of 0.1 to 1 μm and that of the thick film electrodes 14 in the range of 3 to 20 μm. The thick film electrodes 6 are for better electrodes' joining to lead wires, and they are not necessarily needed from the viewpoint of the gas sensor's construction.

The metallic oxide thin film 14 is equivalent to the gas sensitive body of the present invention, and the catalytically active protective layer 15 to the catalytically active protective layer of the present invention.

Then the method of producing gas sensors in accordance with this embodiment will be described.

A pair of thin film electrodes 2 of 0.3 μm thickness, as a first layer, is formed in the steps of: applying paste of organometallic compound of gold to an alumina substrate 0.4 mm thick by the screen coating method; drying the applied paste; and firing the same at 800°C.

Then, a pair of thick film electrodes 6 of 5 μm thickness, as a second layer, is formed in the steps of: applying a thick film forming paste of gold to the thin film electrodes 2, as a first layer, by the screen coating method; drying the applied paste; and firing the same at 800°C.

A gas sensitive film 13 is formed in the steps of: forming a metallic oxide thin film 14; and forming a catalytically active protective layer 15 in such a manner as to be impregnated into the above metallic oxide thin film 14.

The metallic oxide thin film 14 can be formed in the following manner.

First, palladium chloride dihydrate (Chemical Formula 1), as an activator, is weighed in a 100 ml beaker so that the value of (Formula 1) becomes 1% by mol, and 16 g of butyl carbitol and 8 g of butyl carbitol acetate are added and stirred for a while. Then, 8 g of polyvinylpyrrolidinone as a viscosity modifier is added to the mixture, and 24 g of tin 2-ethylhexanoate (Chemical Formula 2) was added and stirred/mixed to obtain a composition for forming the desired metallic oxide thin film 14.

(Formula 1)

$$Pd/(Sn + Pd) \times 100$$

(Chemical Formula 1)

$PdCl_2 \cdot 2H_2O$

(Chemical Formula 2)

$Sn(OOCCH(CH_2CH_3)(CH_2)3CH_3)_2$

The composition for forming the metallic oxide thin film 14 is applied on an alumina substrate 0.4 mm thick by the screen printing method and fired at 700°C for 1 hour to form the metallic oxide thin film 14 of 2100 nm thickness whose main constituent is tin oxide.

After the formation of the film for forming the metallic oxide thin film 14 on the substrate 1, the film is fired at several hundred degrees or more to form the metallic oxide thin film 14. For the application of the composition for forming the metallic oxide thin film 14, the methods such as screen printing, roll coating, dip coating and spin coating can be used; however, the screen coating method is preferably used. As the firing temperature, any temperature can be employed as long as it is not less than the temperature which permits the decomposition of the composition for forming the metallic oxide thin film 14 and not more than the temperature which permits the deformation of the substrate 1, but preferably the temperature is in the range of 400 to 800°C.

The composition for forming the metallic oxide thin film 14 is synthesized in the following manner.

First, an organic solvent is added to an activator to dissolve it. The activator is a metallic salt that is added to improve the sensitivity and gas selectivity as the gas sensitive body, and concrete examples of the activators applicable are alkaline earth metallic salts such as metallic magnesium salts, metallic calcium salts, metallic strontium salts and metallic barium salts; transition metallic salts such as metallic titanium salts, metallic zirconium salts, metallic vanadium salts, metallic chromium salts, metallic manganese salts, metallic iron salts, metallic cobalt salts, metallic nickel salts and metallic copper salts; metallic zinc salts; metallic lead salts; metallic cadmium salts; metallic antimony salts; metallic bismuth salts; and metallic palladium salts. As the metallic salt compound, any compound can be used as long as it is relatively stable at room temperature, but easily decomposed by heat treatment, and it does notmatter whether it is an inorganic salt or an organic salt. The inorganic salts applicable include, for example, nitrates, sulfates and chlorides. And the organic salts applicable include, for example, carboxylates, dicarboxylates and acetylacetonato complex salts. As the organic solvent, any organic solvent can be used as long as it can dissolve both metallic tin soap and a viscosity modifier. The organic solvents applicable include, for example, ether alcohols such as methoxyethanol and butyl carbitol; β-diketones such as acetylacetone; esters such as butyl carbitol acetate; and terpene-based solvents such as α-terpineol.

Then, a viscosity modifier is added to and mixed with the above organic solution. As the viscosity modifier, any polymer can be used as long as it produces a thickening effect of increasing the viscosity of the organic solution. Such viscosity modifies include, for example, polyvinylpyrrolidinone and ethyl cellulose.

Lastly, metallic tin soap is added to and mixed with the above organic solution. The metallic tin soap applicable includes, for example, tin 2-ethylhexanoate and tin naphthenate.

As the metallic oxide thin film 14, any thin film can be used as long as it is a metallic oxide semiconductor thin film, and concrete examples of the metallic oxide semiconductor thin films are thin films of tin oxide, indium oxide, tungsten oxide and zinc oxide. The process of producing the thin film is not limited to thermal decomposition method of the aforementioned organometallic compounds, but chemical film forming processes, such as sol-gel process and CVD process, and physical film forming processes, such as vacuum evaporation coating process and sputtering process, can also be used.

The catalytically active protective layer 15 can be formed in the following manner.

Ten grams of tetraethyl orthosilicate is dissolved in 8 g of ethanol to prepare a raw material solution, 0.2 g of concentrated hydrochloric acid and 10 g of water are added to 8 g of ethanol to prepare a hydrolysis solution, and the hydrolysis solution is dropped into and mixed with the above raw material solution at room temperature to obtain a composition for forming the catalytically active protective layer 15.

A 10-fold dilution of the composition for forming the catalytically active protective layer 15 is prepared with ethanol, impregnated into the metallic oxide thin film containing tin oxide as a main constituent by the dip coating method, dried at 60°C for 5 minutes, and fired at 300°C for 5 minutes and subsequently at 750°C for 20 minutes. This operation is performed twice to form a catalytically active protective layer 15 made up of silicon oxide. The water absorption rate of the catalytically active protective layer 15 is 4%.

The catalytically active protective layer 15 is formed by impregnating the composition for forming the catalytically active protective layer 15 into the metallic oxide thin film 14 and firing the same at a temperature of several hundred degrees or higher. Keeping the water absorption rate of the catalytically active protective layer 15 at 5% or less prevents the formation of hydroxyl groups which are the cause of deterioration in the gas sensitive body. For the application of the composition for forming the catalytically active protective layer 15, methods such as screen printing, roll coating, dip coating and spin coating can be used; however, the dip coating or spin coatingmethod is preferable. As the firing temperature, any temperature can be employed as long as it is not less than the temperature which permits the decomposition of the composition for forming the catalytically active protective layer 15 and not more than the temperature which permits the deformation of the substrate 1, and preferably the temperature is in the range of 400 to 800°C.

In more particular, the composition for forming the catalytically active protective layer 15 is synthesized in the following manner.

First, an organosilicon compound is added to and dissolved in an organic solvent to obtain a raw material solution.

As the organosilicon compound, any organosilicon compound can be used as long as it undergoes hydrolysis/polycondensation, and concrete examples applicable are tetraalkoxysilanes such as tetraethyl orthosilicate, fluoroalkyltrialkoxysilane such as fluoromethyltriethyl silicate, and metal alkoxide such as difluoroalkyldialkoxysilane. Then, a predetermined amount of water, as the hydrolysis solution, and an acid such as hydrochloric acid, nitric acid or sulfuric acid, as a catalyst for the hydrolysis, are dissolved in an organic solvent. A promoter can also be added to this organic solvent. The promoter is a metallic salt added to improve the catalytic activity of the catalytically active protective layer 15 and the reliability and gas selectivity of the gas sensitive body, and concrete examples of the promoters applicable are transition metallic salts such as metallic titanium salts, metallic vanadium salts, metallic chromium salts, metallic manganese salts, metallic iron salts, metallic cobalt salts, metallic nickel salts, metallic zinc salts, metallic copper salts, and noble metallic salts such as metallic platinum salts, metallic palladium salts, metallic ruthenium salts and metallic rhodium salts. As the compound, any compound can be used as long as it is dissolved in an acidic solution and exists stably, and it does not matter whether it is an inorganic salt or an organic salt. Theinorganicsaltsapplicableinclude, for example, nitrates, sulfates and chlorides. And the organic salts applicable include, for example, carboxylates, dicarboxylates and acetylacetonato complex salts. As the organic solvent, considering the reactivity with the alkoxyl groups of the organosilicon compound, organic solvents having the same functional groups, in particular, alcohols, such as methanol and ethanol, and ether alcohols, such as methoxyethanol and ethoxyethanol, can be used.

Lastly, a predetermined amount of water and acid, and, depending on the situation, a hydrolysis solution containing a promoter are dropped into and mixed with the raw material solution in which the organosilicon compound has been dissolved to obtain a composition for forming the catalytically active protective layer 15. During or after the hydrolysis, heat may be applied.

Finally, gas sensitivity to 1 ppm dimethyl sulfide was measured using the sensor elements produced. Each sensor element was fixed in a 7-liter acrylic box, while the temperature of the element was controlled to 350°C. The resistance of each sensor element was measured when leaving it in the air in the acrylic box and when introducing 10 ml of 700 ppm dimethyl sulfide into the acrylic box to bring it into contact with 1 ppm dimethyl sulfide, so that the change in resistance of the sensor element was determined. The value RG/RA was obtained as the sensitivity of each sensor, where RA represented the resistance of each sensor element in the air and RG that of each sensor element 30 minutes after the gas containing 1 ppm dimethyl sulfide was introduced into the acrylic box. The resultant sensor sensitivity to 1 ppm dimethyl sulfide was 0.60 for the sensor element that included the above catalytically active protective layer 15 and 0.90 for the sensor element without the catalytically active protective layer 15.

The catalytically active protective layer 15 serves to decompose alcohols, aldehydes, mercaptans and amines, which are the cause of deterioration in the sensor's durability, or to prevent water content from coming in direct contact with the surface of the metallic oxide particles, which are the skeleton of the gas sensitive body. In more particular, the inventors of this invention considers that the catalytically active protective layer 15 serves to prevent (1) the reduction of the catalyst, as the cause of the deterioration in catalyst, in the electrodes and the gas sensitive body, which play a key role of the sensor, by the reducing gases of alcohols, aldehydes, etc. generated from vegetables and fruits, (2) the chemical adsorption of mercaptans, amines, etc. on the electrode surface and the catalyst, and (3) the formation of hydroxyl groups on the surface of the gas sensitive film due to the water in the air. And it is, of course, considered that the presence of such a catalytically active protective layer contributes to the activation of the metallic oxide particles constituting the skeleton of the gas sensitive body, and hence the improvement of its responsiveness to gases to be sensed.

So far, embodiments 1 and 2 of the present invention have been described.

In the following, several examples of the present invention will be described in further detail with reference to the accompanying drawings.

(Example 1)

Paste of organometallic compound of gold was applied on an alumina substrate 0 . 4 mm thick by the screen printing method, dried, and fired at 800°C to form thin film electrodes 2 of 0.3 μm thickness, as a first layer.

Then, thick film printing paste of gold was applied on the thin film electrodes 2 as a first layer by the screen printing method, dried, and fired at 800°C to form thick film electrodes 6 of 6 μm thickness, as a second layer.

First, each metal (M = Mn, Fe, Ni, Co, Zn) 2-ethylhexanoate, as an activator, was weighed in a 100 ml beaker so that the value of (Formula 2) became 1% by mol, then 10 g of butyl carbitol solution in which 1 g of ethyl cellulose, as a viscosity modifier, had been dissolved was added, 9 g of tin 2-ethylhexanoate (Chemical Formula 2) was also added, and the solution was stirred/mixed to obtain a composition for forming a desired gas sensitive body.

(Chemical Formula 2)

$$Sn(OOCCH(CH_2CH_3)(CH_2)_3CH_3)_2$$

(Formula 2)

$$M/(Sn+M) \times 100$$

Each composition for forming the gas sensitive body was applied on an alumina substrate 0.4 mm thick by the screen printing method and fired at 700°C for 1 hour to form a gas sensitive film of 2400 nm thickness made up of metallic oxide whose main constituent was tin oxide.

Then 10 g of tetraethyl orthosilicate was dissolved in 23 g of ethanol to prepare a raw material solution, 0.2 g of concentrated hydrochloric acid and 3 g of water were added to 23 g of ethanol to prepare a hydrolysis solution, and the hydrolysis solution was dropped into and mixed with the raw material solution at room temperature to obtain a composition for forming a catalytically active protective layer.

The composition for forming the catalytically active protective layer was applied, by the dip coating method, on the gas sensitive film made up of a metallic oxide whose main constituent is tin oxide, fired at 500°C for 20 minutes to form the catalytically active protective layer of silicon oxide 200 nm thick.

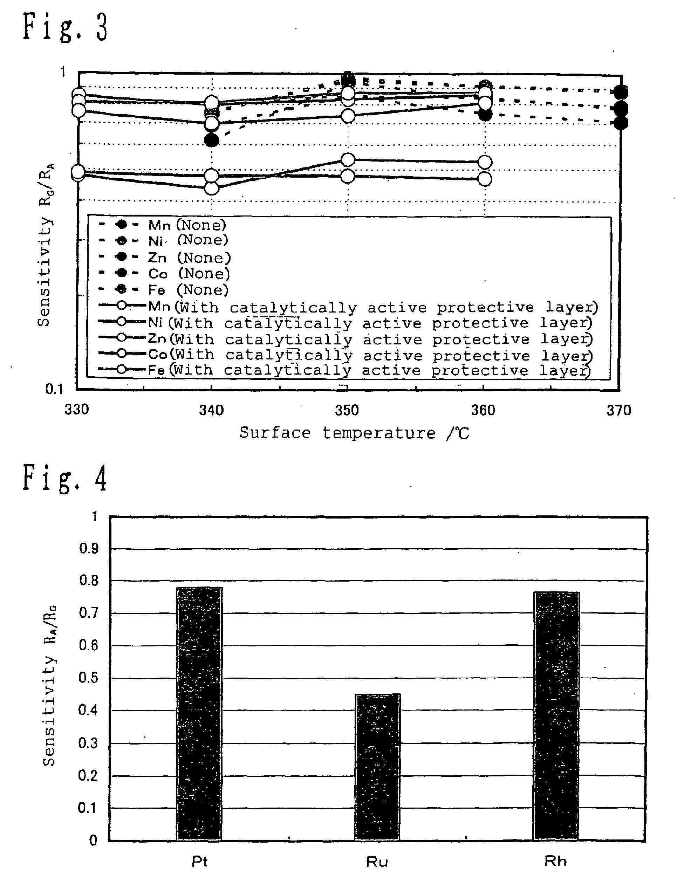

Finally, gas sensitivity to 1 ppm dimethyl sulfide was measured using the sensor elements produced. Each sensor element was fixed in a 7-liter acrylic box, while the temperature of the element was controlled to 330 to 370°C. The resistance of each sensor element was measured when leaving it in the air in the acrylic box and when. introducing 10 ml of 700 ppm dimethyl sulfide into the acrylic box to bring it into contact with 1 ppm dimethyl sulfide, so that the change in resistance of the sensor element was determined. The value RG/RA was obtained as the sensitivity of each sensor, where RA represented the resistance of each sensor element in the air and RG that of each sensor element 30 minutes after the gas containing 1 ppm dimethyl sulfide was introduced into the acrylic box. The resultant sensor sensitivity of each sensor element was shown in Figure 3. The sensitivity to 10 ppm of acetaldehyde was 0.50 for the sensor elements that included the above catalytically active protective layer 15 and 0.70 for the sensor elements without the catalytically active protective layer 15.

(Example 2)

A gas sensitive film made up of a metal oxide whose main constituent was tin oxide was formed using iron 2-ethylhexanoate, as an activator, in such an amount that the above value of (Formula 1) was 1% by mol.

Then 10 g of tetraethyl orthosilicate was dissolved in 23 g of ethanol to prepare a raw material solution, 0.2 g of concentrated hydrochloric acid and 3 g of each noble metallic salt (ruthenium nitrate, rhodium chloride and platinum-P salt) were added to 23 g of ethanol to prepare hydrolysis solutions, and each of the hydrolysis solution was dropped into and mixed with the raw material solution at room temperature to obtain a composition for forming a catalytically active protective layer.

The gas sensitivity to 1 ppm dimethyl sulfide was measured using the sensor elements produced, while keeping the temperature of each element at 350°C. The resultant sensor sensitivity of each sensor element was shown in Figure 4.

(Example 3)

First, palladium chloride (Chemical Formula 1) , as an activator, was weighed in a 100 ml beaker so that the value of (Formula 1) became 1% by mol, 13 g of butyl carbitol solution, in which 1 g of ethyl cellulose as a viscosity modifier was dissolved, was added to the beaker, and 6 g of tin 2-ethylhexanoate (Chemical Formula 3) was also added and stirred/mixed to obtain a composition for forming a desired gas sensitive body.

(Formula 1)

$$Pd/(Sn + Pd) \times 100$$

(Chemical Formula 1)

$$PdCl_2 \cdot 2H_2O$$

The composition for forming the gas sensitive body was applied on an alumina substrate 0.4 mm thick by the screen printing method and fired at 700°C for 1 hour to form the gas sensitive film 1800 nm thick made up of a metallic oxide whose main constituent is tin oxide.

Then 10 g of tetraethyl orthosilicate was dissolved in 8 g of ethanol to prepare a raw material solution, 0.2 g of concentrated hydrochloric acid and 10 g of water were added to 8 g of ethanol to prepare a hydrolysis solution, and the hydrolysis solution was dropped into and mixed with the raw material solution at room temperature to obtain a composition for forming a catalytically active protective layer.

Then, gas sensitivities to 1 ppm ethylene, ethanol, ammonia and dimethyl sulfide were measured using the sensor element produced, while keeping the temperature of the sensor element at 350°C. The resultant values of the sensor sensitivity to the above gases were 0.60, 0.12, 0.80 and 0.62, respectively.

(Example 4)

Ten grams of tetraethyl orthosilicate was dissolved in 23 g of ethanol to prepare a raw material solution, 0.2 g of concentrated hydrochloric acid and 3 g of water were added to 23 g of ethanol to prepare a hydrolysis solution, and the hydrolysis solution was dropped into and mixed with the raw material solution at room temperature to obtain a composition for forming a catalytically active protective layer.

Five- to fifteen-fold dilutions of the composition for forming the catalytically active protective layer were prepared. Each dilution of the composition for forming the catalytically active protective layer was impregnated into the gas sensitive film made up of a metallic oxide whose main constituent was tin oxide by the dip coating and fired at 500°C for 20 minutes to form a catalytically active protective layer 200 nm thick made up of silicon oxide.

The others were the same as example 1.

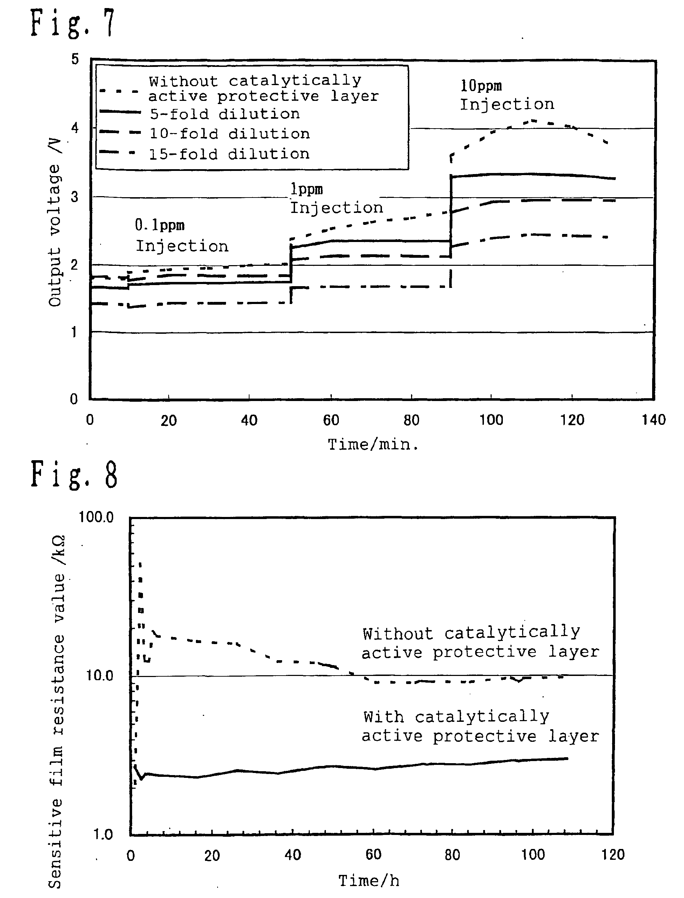

The gas responsive characteristics to 0.1 ppm, 1 ppm, and 10 ppm dimethyl sulfide were measured using the sensor element produced, and the measures results are shown in Figure 7.

(Example 5)

A 5-fold dilution of the composition for forming the catalytically active protective layer were prepared, and the dilution was impregnated into the metallic oxide thin film whose main constituent was tin oxide by the dip coating, dried for at 60°C 5 minutes, and fired at 300°C for 5 minutes and subsequently at 750°C for 20 minutes. These operations were performed twice to form the catalytically active protective layer 5 made up of silicon oxide. The others were the same as example 1.

The changes in resistance of the sensor elements when leaving them at 40°C, 95% RH for 100 hours are shown in Figure 8. The figure reveals that the resistance of the sensor element of the present invention that included the catalytically active protective layer hardly changed even after 100 hours elapsed.

So far, examples 1 to 5 of the present invention have been described in detail.

As is evident from the description so far, the catalytically active protective layer containing silicon oxide as a main constituent and formed on and/or in the aforementioned gas sensitive film serves to decompose alcohols, aldehydes, mercaptans and amines, the causes of deterioration in gas sensors, or inhibit them from entering the gas sensitive film; it becomes possible to prevent the reduction of the catalyst by the reducing gases of alcohols and aldehydes generated from vegetables and fruits and the chemical adsorption of mercaptans and amines on the surface of the electrodes and catalysts, which are the causes of the deterioration in the electrodes and the catalyst in the gas sensitive body that play a key role of the gas sensors. The catalytically active protective layer also produces the effect of preventing the formation of hydroxyl groups on the surface of the gas sensitive film due to the water in the air.

According to the present invention, a catalytically active protective layer containing silicon oxide as a main constituent is formed on a gas sensitive film by the sol-gel process, as a chemical film forming method, or impregnated into the gaps of metallic oxide particles that constitute the skeleton of the gas sensitive body, whereby a gas sensor can be obtained which has a gas sensitive film of high adhesion, is excellent in thermal shock resistance during its operation, and is highly reliable. Further, a method is employed, in which a catalytically active protective layer is formed in the steps of: preparing an organic solution by dropping/mixing an organic solution containing a promoter and water into/with an organic solution of organosilicon compound and hydrolyzing/polycondensing the mixed solution; applying the organic solution on the gas sensitive film and drying, and firing the same. Thismakes it easy to allow a transition metal element or a noble metal element, as a promoter, to be uniformly dispersed in and added to the catalytically active protective layer, resulting in production of a more highly active catalytically active protective layer. Thus, a gas sensor of high reliability can be obtained which senses gases as objects of sensing (ethylene, ethanol, aldehydes, mercaptans and amines) at a sensitivity level of as high as 1 ppm.

Industrial Applicability

The present invention offers the advantage of being capable of sensing gases of, for example, ethylene, ethanol, aldehydes, mercaptans and amines, which are important in sensing the freshness of vegetables and fruits, with satisfactory responsiveness and excellent durability.

**Claims**

**1.** A gas sensor, comprising:

an insulating substrate;
a pair of first electrodes provided on the substrate in such a manner as to leave a fixed space between them;
a gas sensitive body including a metallic oxide, which is provided in such a manner as to substantially fill at least the fixed space; and

a catalytically active protective layer formed in such a manner as to cover the surface of the metallic oxide exposed to the outside and/or to make up for the gaps exiting inside the metallic oxide.

**2.** The gas sensor according to claim 1, wherein the first electrodes are thin film electrodes,

further comprising a pair of thick film electrodes for applying a voltage, provided on the respective thin film electrodes.

**3.** The gas sensor according to claim 2, wherein the thick film electrodes are provided in such a manner as not to come in contact with the gas sensitive body.

**4.** The gas sensor according to claim 3, wherein the catalytically active protective layer is formed in such a manner as to also cover the thick film electrodes.

**5.** The gas sensor according to claim 1, wherein the metallic oxide contains, as a main constituent, at least one of tin oxide, indium oxide, zinc oxide and tungsten oxide.

**6.** The gas sensor according to claim 1, wherein the catalytically active protective layer contains silicon oxide as a main constituent and at least one of titanium, vanadium, chromium, manganese, iron, cobalt, nickel, zinc, copper, platinum, palladium, ruthenium and rhodium, as a promoter.

**7.** A method of producing the gas sensor according to claim 1, comprising a step of forming the catalytically active protective layer using the sol-gel process.

**8.** A method of producing the gas sensor according to claim 1, comprising a step of forming the catalytically active protective layer using a solution obtained by: dropping and mixing an organic solution containing a promoter, water and an acid into an organic solution containing an organosilicon compound; and hydrolyzing and polycondensing.

**9.** The method of producing a gas sensor according to claim 8, wherein the organosilicon compound contains at least one of tetraalkoxysilane, fluoroalkyltrialkoxysilane and difluoroalkyldialkoxysilane.

**10.** The method of producing a gas sensor according to claim 8, wherein the promoter is any one of nitrate complex salts, chloride complex salts, sulfate complex salts and acetylacetonato complex salts which contains at least one metal of titanium, vanadium, chromium, manganese, iron, cobalt, nickel, zinc, copper, platinum, palladium, ruthenium and rhodium.

**11.** The method of producing a gas sensor according to claim 8, wherein the acid is any one of hydrochloric acid, sulfuric acid, nitric acid, ammonia and alcohol amines.

**12.** The method of producing a gas sensor according to claim 8, wherein the organic solution containing the organosilicon compound further contains an organometallic compound for accelerating the polycondensation.

**13.** The method of producing a gas sensor according to claim 12, wherein the organometallic compound is metallic alkoxide containing at least one metal of titanium, zirconium, tin, aluminum and zinc.

Fig. 1

3
5
1
2

Fig. 2

6
5
3
1
2

Fig. 3

1
0.1
330
340
350
360
370
Sensitivity $R_G/R_A$
Surface temperature /℃
Mn (None)
Ni (None)
Zn (None)
Co (None)
Fe (None)
Mn (With catalytically active protective layer)
Ni (With catalytically active protective layer)
Zn (With catalytically active protective layer)
Co (With catalytically active protective layer)
Fe (With catalytically active protective layer)


Fig. 4

1
0.9
0.8
0.7
0.6
0.5
0.4
0.3
0.2
0.1
0
Sensitivity $R_A/R_G$
Pt
Ru
Rh

Fig. 5

15
14
13
1
2

Fig. 6

15
14
6
13
1
2

Without catalytically active protective layer
5-fold dilution
10-fold dilution
15-fold dilution
0.1ppm Injection
1ppm Injection
10ppm Injection
Output voltage /V
Time/min.
0
1
2
3
4
5
0
20
40
60
80
100
120
140

Fig. 8

Without catalytically active protective layer
With catalytically active protective layer
Sensitive film resistance value /kΩ
Time/h
1.0
10.0
100.0
0
20
40
60
80
100
120

Fig. 9

7
8
1

## INTERNATIONAL SEARCH REPORT

International application No. PCT/JP02/11819

**A. CLASSIFICATION OF SUBJECT MATTER**

Int.Cl[7] G01N27/327

According to International Patent Classification (IPC) or to both national classification and IPC

**B. FIELDS SEARCHED**

Minimum documentation searched (classification system followed by classification symbols)

Int.Cl[7] G01N27/327

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| | JP 4-145354 A (Figaro Engineering Inc.), 19 May, 1992 (19.05.92), | |
| X | Page 2, upper right column, line 11 to lower right column, line 11; Figs. 20, 21 | 1-6 |
| Y | Page 2, upper right column, line 11 to lower right column, line 11; Figs. 20, 21 (Family: none) | 7-13 |
| | JP 2000-180397 A (Honda Motor Co., Ltd.), 30 June, 2000 (30.06.00), | |
| X | Par. No. [0020]; Fig. 4 | 1-6 |
| Y | Par. No. [0020]; Fig. 4 (Family: none) | 7-13 |

[X] Further documents are listed in the continuation of Box C. [ ] See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 21 February, 2003 (21.02.03) | 11 March, 2003 (11.03.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/11819

C (Continuation). DOCUMENTS CONSIDERED TO BE RELEVANT

| Category* | Citation of document, with indication, where appropriate, of the relevant passages | Relevant to claim No. |
|---|---|---|
| | JP 10-82755 A (LG Electronics Inc.), 31 March, 1998 (31.03.98), | |
| X | Par. Nos. [0010] to [0013]; Figs. 3, 4 | 1-6 |
| Y | Par. Nos. [0010] to [0013]; Figs. 3, 4 & CN 1178903 A & KR 166931 B | 7-13 |
| Y | JP 2001-225410 A (Oji Paper Co., Ltd.), 21 August, 2001 (21.08.01), Par. No. [0022] (Family: none) | 7-13 |

Form PCT/ISA/210 (continuation of second sheet) (July 1998)